(19) Europäisches Patentamt
European Patent Office
Office européen des brevets

(11) Veröffentlichungsnummer: **0 400 436 A2**

(12) # EUROPÄISCHE PATENTANMELDUNG

(21) Anmeldenummer: **90109561.2**

(22) Anmeldetag: **19.05.90**

(51) Int. Cl.5: **C08G 65/20, C08G 65/32, C07C 43/15**

Die Bezeichnung der Erfindung wurde geändert (Richtlinien für die Prüfung im EPA, A-III, 7.3).

(30) Priorität: **30.05.89 DE 3917457**

(43) Veröffentlichungstag der Anmeldung: **05.12.90 Patentblatt 90/49**

(84) Benannte Vertragsstaaten: **BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Hickmann, Eckhard, Dr.**
**Kantstrasse 23**
**D-6701 Dannstadt-Schauernheim(DE)**

(54) **Polytetrahydrofuranether.**

(57) Polytetrahydrofuranether der allgemeinen Formel

$R^1 \{O-(CH_2)_4\}_n O-R^2$   I,

in der n für eine Zahl von 3 bis 70 steht und $R^1$ und $R^2$ Kohlenwasserstoffreste, die 3 bis 5 Kohlenstoffatome und eine Doppelbindung enthalten, bedeuten, wobei $R^2$ jedoch auch für ein Wasserstoffatom stehen kann.

EP 0 400 436 A2

## Polyethertetrahydrofuranether

Diese Erfindung betrifft neue Polytetrahydrofuranether der allgemeinen Formel
$R^1\{O-(CH_2)_4\}_n O-R^2$ I,
in der n für eine Zahl von 3 bis 70 steht und $R^1$ und $R^2$ Kohlenwasserstoffreste, die 3 bis 5 Kohlenstoffatome und eine Doppelbindung enthalten, bedeuten, wobei $R^2$ jedoch auch für ein Wasserstoffatom stehen kann.

Die neuen Polyethertetrahydrofurane der Formel I sind wertvolle Bausteine für Polymere.

Als Kohlenwasserstoffreste $R^1$ und $R^2$ enthalten die Polytetrahydrofuranether z.B. Reste der Formeln $CH_2=CH-CH_2-$, $CH_3CH=CH-$, $CH_3-C(CH_3)=CH$, $CH_2=C(CH_3)-CH_2-$ und $CH_3-CH=CH-CH_2-$.

Die neuen Polytetrahydrofuranether haben z.B. die Formel

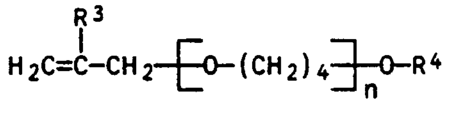
II,

in der $R^3$ ein Wasserstoffatom oder die Methylgruppe, $R^4$ ein Wasserstoffatom oder den Rest der Formel $-CH_2-C(R^3)=CH_2$ und n eine Zahl von 3 bis 70 bedeuten oder die Formel

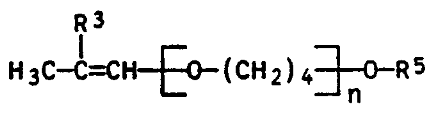
III,

in der $R^3$ ein Wasserstoffatom oder die Methylgruppe, $R^5$ ein Wasserstoff oder den Rest der Formel $-CH=C(R^3)-CH_3$ und n eine Zahl von 3 bis 70 bedeuten.

Polytetrahydrofurane der allgemeinen Formel
$H\{O(CH_2)_4\}_n OH$ IV,
(im folgenden PTHF genannt) werden z.B. durch kationische Polymerisation von Tetrahydrofuran (im folgenden THF genannt) hergestellt. In der Strukturformel IV gibt der Polymerisationsgrad n die Anzahl der vom THF abgeleiteten Oxi-butan-1,4-diyl-Einheiten pro Molekül an; er liegt üblicherweise bei n = 3 (entsprechend einem mittleren Molgewicht von $\overline{MG}$ = 234) bis n etwa 70 (entsprechend einem $\overline{MG}$ von etwa 5000). Der Polymerisationsgrad n bzw. das diesem Wert entsprechende mittlere Molgewicht $\overline{MG}$, durch die die bei der Polymerisation von THF erhältlichen PTHF-Gemische charakterisiert werden, können z.B. durch osmometrische oder titrimetrische Analysen ermittelt werden. Technisch besonders interessant sind z.B. die folgenden PTHF-Gemische: PTHF 250 ( $\overline{MG}$ = ca 250, $\overline{n}$ = ca 3), PTHF 650 ( $\overline{MG}$ = ca 650, $\overline{n}$ = ca 9), PTHF 1000 ( $\overline{MG}$ = ca 1000, $\overline{n}$ = ca 14), PTHF 2000 ( $\overline{MG}$ = ca 2000, $\overline{n}$ = ca 27), PTHF 2900 ( $\overline{MG}$ = ca 2900, $\overline{n}$ = ca 40) und PTHF 4500 ( $\overline{MG}$ = ca 4500, $\overline{n}$ = ca 62).

Jedes dieser PTHF-Gemische enthält ein breites Spektrum von PTHF-Homologen, deren Anzahl ca 10 bis 20 im niedrigen Molmassenbereich beträgt und auf über 30 im hohen Molmassenbereich ansteigt.

PTHF wird als $\alpha$, $\omega$-Diol für die Herstellung von Polymeren verwendet. Hierbei zeichnet sich PTHF wegen seiner wertvollen Eigenschaften als Baustein für elastomere und thermoplastische Polymere aus (P. Dreyfuss "Handbook of Elastomers, New Developments and Technology", 1988, S. 695).

Als $\alpha$, $\omega$-Diol ist die Reaktionsfähigkeit des PTHF allerdings auf die typischen Reaktionen von primären Alkoholen beschränkt. Es hat daher nicht an Versuchen gefehlt, dem PTHF durch Modifizieren der Endgruppen eine andere Reaktivität zu verleihen, um dadurch seine Verwendungsmöglichkeiten zu erweitern. So liefert z.B. die Umsetzung des PTHF mit Diisocyanaten im Molverhältnis 1 : 2 PTHF-Diurethane mit freien, endständigen Isocyanatgruppen, und durch Umesterung von (Meth-)Acrylsäureestern mit PTHF erhält man PTHF-bis(meth-)acrylate. Bei derartigen Umsetzungen mit Gemischen von PTHF-Homologen erhält man wiederum Gemische von homologen PTHF-Derivaten, die sich strukturell ebenfalls nur durch die unterschiedliche Zahl der wiederkehrenden Oxi-butan-1,4-diyl-Einheiten in der Polyetherkette unterscheiden.

Gegenstand dieser Erfindung sind nun die neuen Polytetrahydrofuranether der allgemeinen Formel I. Man stellt sie z.B. nach an sich bekannten Methoden (s. Houben-Weyl, "Methoden der organischen Chemie", Band 6/3, S. 23-36) dadurch her, daß man a) Polytetrahydrofurane der allgemeinen Formel
$H\{O-(CH_2)_4\}_n OH$ IV,

in der n die oben genannte Bedeutung hat, mit Verbindungen der allgemeinen Formel

$R^1$-X V,

in der $R^1$ die obengenannte Bedeutung hat, X ein Halogenatom oder einen Rest der Formel $-SO_2-R^6$ und $R^6$ einen Alkylrest mit 1 bis 6 C-Atomen bedeuten, umsetzt oder daß man b) Polytetrahydrofuranderivate der Formel

$X-(CH_2)_4-O[(CH_2)_4-O-]_{n}-(CH_2)_4-X$ VI,

in der X und n die obengenannte Bedeutung haben, mit Alkoholen der Formel

$R^1$-OH VII,

in der $R^1$ die obengenannte Bedeutung hat, umsetzt. Bei diesen Umsetzungen wendet man die Reaktionspartner z.B. in einem Molverhältnis von 2:1 bis 1:2 an. Die Umsetzung wird z.B. bei Temperaturen bis 100 °C, vorzugsweise 0 bis 80 °C und in Gegenwart von basisch wirkenden Stoffen durchgeführt. Als basisch wirkende Stoffe kommen vorzugsweise tertiäre Amine, z.B. Trialkylamine, wie Tributylamin, Triethylamin, N,N-Dimethylanilin oder heterocyclische Stickstoffverbindungen mit tertiärem N-Atom, wie Pyridin, Picolin und Chinolin in Betracht. Man kann als basisch wirkende Stoffe aber auch Carbonate, Hydroxide oder Hydride der Alkali- oder Erdalkalimetalle, wie Kaliumcarbonat, Kaliumhydroxid, Natriumhydrid oder Alkoholate, wie Kalium-tertiär-butoxylat einsetzen.

Man setzt die basisch wirkenden Stoffe z.B. in stöchiometrischen Mengen, bezogen auf die Verbindung V bzw. VI, ein, wobei jedoch auch mit einem über- oder Unterschuß von 10 bis 20 % gearbeitet werden kann.

Man führt die Umsetzung zweckmäßigerweise in einem Lösungsmittel durch. Geeignet sind alle Lösungsmittel, die unter den Umsetzungsbedingungen nicht mit den Reaktionsteilnehmern reagieren und in denen PTHF bzw. das PTHF-Derivat löslich ist, das sind z.B. Ether, wie Methyl-tert.-butylether (MTBE), Diethylether, Dioxan oder Tetrahydrofuran, Carbonylverbindungen, wie Aceton, Methylethylketon, Methylisobutylketon oder Essigester, aliphatische Kohlenwasserstoffe, wie Cyclohexan, Methylenchlorid oder Dichlorethan, oder aromatische Kohlenwasserstoffe, wie Toluol, Benzol oder Xylol. Man kann die Umsetzung aber auch in den obengenannten tertiären Aminen durchführen.

Die Umsetzungsgemische werden auf einfache Weise z.B. dadurch aufgearbeitet, daß man Wasser hinzugibt, die organische Phase abtrennt, zur Abtrennung gebildeter Salze und gegebenenfalls des Lösungsmittels mit Wasser wäscht und restliches Lösungsmittel abdestilliert.

Die neuen Polytetrahydrofuranether der Formel III kann man auch aus den entsprechenden Alkylethern der Formel II durch eine an sich bekannte basenkatalysierte Isomerisierung (J. Amer. Chem. Soc., 1961, 83, 1701-1704 und 1773) gewinnen. Man isomerisiert z.B. bei Temperaturen von 130 bis 180 °C in Gegenwart von basisch wirkenden Stoffen, wie Alkalimetallalkoholaten, insbesondere Kalium-tert.-butanolat und Natrium-tert.-amylat.

Die erfindungsgemäßen PTHF-Derivate werden als Comonomere in Polyolefinen und Polyvinylethern sowie als Vernetzer in hydroxylgruppenhaltigen Polymeren, u.a. in Dispersionen, verwendet.

Zur Identifizierung und Gehaltsbestimmung der erfindungsgemäßen PTHF-Ether eignet sich in besonderem Maße die $^1$H-NMR-Spektroskopie.

## Beispiel 1

Herstellung von PTHF 650-bisallylether:

Das Gemisch aus 520 g PTHF 650, 2,4 l Toluol, 536 g KOH-Pulver und 3 g Triethylamin wird unter Rühren bei 60-65 °C innerhalb von 3 h mit 305 g Allylchlorid versetzt. Das Reaktionsgemisch läßt man 4,5 h nachreagieren, rührt es dann mit 600 ml Wasser aus und wäscht die organische Phase mit 200 ml Wasser nach. Nach Abziehen der Niedersieder am Rotationsverdampfer und Trocknen des Rückstandes im Ölpumpenvakuum bei 80 °C erhält man 579 g PTHF 650-bisallylether ( $\bar{n}$ = 9,0).

Das Produkt zeigt im NMR-Spektrum die für die Allyloxigruppen typischen Multipletts bei $\delta$ = 3,9-4,0-(2x2 H), 5,1-5,3(2x2 H) und 5,8-6,0 (2x1 H). Aus den Intensitätsverhältnissen aller NMR-Banden kann eine mittlere Molmasse von 748 abgeleitet werden (berechnet: 730) sowie eine Bromzahl von 42,8 (gemessen: 42). Alkoholgruppen sind analytisch nicht mehr nachweisbar.

## Beispiel 2

Herstellung von PTHF 1000-bisallylether

Das Gemisch aus 100 g PTHF 1000, 300 ml Toluol, 64 g KOH-Pulver und 1,0 g Tetrabutylammoniumbromid wird unter Rühren bei 23-24 °C innerhalb von 1 h mit 22,8 g Allylchlorid versetzt und noch weitere 12 h gerührt. Nach der unter Beispiel 1 beschriebenen Aufarbeitung erhält man 104,9 g PTHF 1000-bisallylether ( $\overline{n}$ = 13,9).

Das Produkt zeigt im NMR-Spektrum die für die Allyloxigruppen typischen Multipletts (s. Beispiel 1). Aus den Intensitätsverhältnissen aller NMR-Banden kann eine mittlere Molmasse von 1097 abgeleitet werden (berechnet: 1 080) sowie eine Bromzahl von 29,2 (gemessen: 30). Alkoholgruppen sind analytisch nicht mehr nachweisbar.

Beispiele 3-9

Die in Tabelle 1 aufgeführten Beispiele 3-11 wurden in Analogie zu Beispielen 1 und 2 ausgeführt:

Tabelle 1

| Beispiel | PTHF ($\overline{MG}$) | n | Menge (g) | Reagenz Formel V | Menge (g) | Base Formel | Base Menge (g) | Lösungsmittel Art | Lösungsmittel Menge (ml) | Reakt.-temp. (°C) | Reakt.-dauer (h) |
|---|---|---|---|---|---|---|---|---|---|---|---|
| 3 | 250 | | 200 | 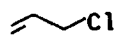 | 305 | KOH<br>$N(C_2H_5)_3$ | 536<br>3 | Toluol | 2400 | 45-62 | 24 |
| 4 | 250 | | 25 | 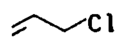 | 16,8 | KOH<br>18-Krone-(6) | 25,6<br>1,05 | THF | 100 | 20-24 | 12 |
| 5 | 650 | | 130 | 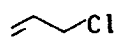 | 38 | NaH | 10,1 | MTBE | 300 | 30-54 | 12 |
| 6 | 1000 | | 100 | 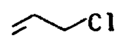 | 19,1 | KOH<br>$N(C_2H_5)_3$ | 48<br>0,3 | Toluol | 300 | 22-26 | 48 |
| 7 | 1000 | | 100 | 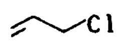 | 22,8 | KOH<br>Aliquat 336 | 64<br>1,2 | Toluol | 300 | 22-27 | 12 |
| 8 | 2000 | | 1000 | 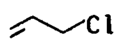 | 191,4 | KOH<br>$N(C_2H_5)_3$ | 335,7<br>1,88 | Toluol | 1500 | 45-50 | 18 |
| 9 | 250 | | 100 | 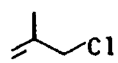 | 181 | KOH<br>$N(C_2H_5)_3$ | 268<br>1,5 | Toluol | 1000 | 60 | 3 |
| 10 | 650 | | 260 | 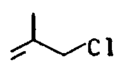 | 181 | KOH<br>$N(C_2H_5)_3$ | 268<br>1,5 | Toluol | 1200 | 60 | 8 |
| 11 | 1000 | | 100 | 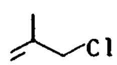 | 27,2 | KOH<br>$(n-C_4H_9)_4NBr$ | 48<br>1,5 | Toluol | 500 | 45-50 | 18 |

Tabelle 1 (Fortsetzung)

| Beispiel | Menge (g) | Produkt $\overline{MG}$ ber. | Produkt $\overline{MG}$ lt. NMR | $\bar{n}$ | Bromzahl lt. NMR | Bromzahl gemessen |
|---|---|---|---|---|---|---|
| 3 | 257 | 330 | 325 | 3,1 | 98,5 | 98 |
| 4 | 28,5 | 330 | 331 | 3,2 | 96,7 | 97 |
| 5 | 138,5 | 730 | 743 | 8,9 | 43,1 | 44 |
| 6 | 104 | 1080 | 1081 | 13,6 | 29,6 | 30 |
| 7 | 107 | 1080 | 1092 | 13,8 | 29,3 | 30 |
| 8 | 1014 | 2080 | 2053 | 27,1 | 15,6 | 16 |
| 9 | 139 | 358 | 367 | 3,3 | 87,2 | 88 |
| 10 | 285 | 758 | 749 | 8,6 | 42,7 | 42 |
| 11 | 107 | 1108 | 1115 | 13,7 | 28,7 | 29 |

Beispiel 12

Herstellung von PTHF 250-bispropenylether

Das Gemisch aus 60,8 g PTHF 250-diallylether (n = 3,2) und 3,0 g Kaliumtert.-butanolat wird unter Stickstoff 3 h auf 150 °C erhitzt. Nach dem Abkühlen versetzt man das Reaktionsgemisch mit je 100 ml Wasser und Methyl-tert.-butylether, neutralisiert es mit Kohlendioxid, trennt die organische Phase ab und wäscht sie mit 100 ml Wasser nach. Nach dem Abziehen des Lösungsmittels und Trocknen des Rückstandes im ölpumpenvakuum bei 80 °C erhält man 58,5 g PTHF 250-bispropenylether ( $\bar{n}$ = 3,3).

Das Produkt zeigt im NMR-Spektrum die für die Propylenoxigruppen typischen Multipletts bei $\delta$ = 1,6-(2x3H), 4,3-4,45(2x1H) und 5,9-6,0(2x1H). Aus den Intensitätsverhältnissen aller NMR-Banden kann eine mittlere Molmasse von 333 abgeleitet werden (berechnet: 330) sowie eine Jodzahl von 152 (gemessen: 150).

Beispiele 13 bis 17

Die in Tabelle 2 aufgeführten Beispiele 13 bis 17 wurden in Analogie zu Beispiel 10 durchgeführt.

Tabelle 2

| Beispiel | eingesetzter PTHF-Ether der Formel II | Menge (g) | KO$^t$BU[2] (g) | Reakt.-temp. (°C) | Reakt.-dauer (h) | erhaltener PTHF-Ether der Formel III | $\bar{n}$ | Menge (g) | $\overline{Mg}$ ber.[1] | lt.NMR | Jodzahl | |
|---|---|---|---|---|---|---|---|---|---|---|---|---|
| | | | | | | | | | | | lt. NMR | gemessen |
| 13 | PTHF 650-diallylether | 115,0 | 3,0 | 150 | 6 | PTHF 650-di-propenylether | 8,9 | 111,4 | 743 | 741 | 68,5 | 68 |
| 14 | PTHF 2000-diallylether | 480 | 6,1 | 145-155 | 5 | PTHF 2000-di-propenylether | 27,3 | 472 | 2053 | 2068 | 24,5 | 25 |
| 15 | PTHF 250-di-(2-methylallylether) | 74,0 | 3,2 | 150 | 6 | PTHF 250-di-(2-methylpropenylether) | 3,1 | 72,1 | 367 | 352 | 144,2 | 146 |
| 16 | PTHF 650-di-(2-methylallylether) | 130,5 | 2,7 | 150 | 3,5 | PTHF 650-di-(2-methylpropenylether) | 8,8 | 125,8 | 749 | 761 | 66,7 | 65 |
| 17 | PTHF 1000-di-(2-methylallylether) | 55,8 | 3,2 | 150-155 | 6 | PTHF 1000-di-(2-methylpropenylether) | 13,4 | 54,0 | 115 | 1092 | 46,5 | 47 |

[1] Mittlere Molmasse des eingesetzten PTHF-(Meth-)Allylethers
[2] Kalium-tert.-butanolat

## Ansprüche

1. Polytetrahydrofuranether der allgemeinen Formel

$R^1 [O-(CH_2)_4]_n O-R^2$ I,

in der n für eine Zahl von 3 bis 70 steht und $R^1$ und $R^2$ Kohlenwasserstoffreste, die 3 bis 5 Kohlenstoffatome und eine Doppelbindung enthalten, bedeuten, wobei $R^2$ jedoch auch für ein Wasserstoffatom stehen kann.

2. Polytetrahydrofuranether der allgemeinen Formel

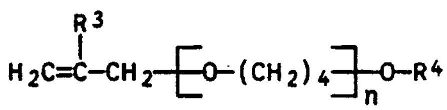

II,

in der $R^3$ ein Wasserstoffatom oder die Methylgruppe, $R^4$ ein Wasserstoffatom oder den Rest der Formel $-CH_2-C(R^3)=CH_2$ und n eine Zahl von 3 bis 70 bedeuten.

3. Polytetrahydrofuranether der allgemeinen Formel

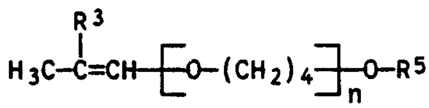

III,

in der $R^3$ ein Wasserstoffatom oder die Methylgruppe, $R^5$ ein Wasserstoffatom oder den Rest der Formel $-CH=C(R^3)-CH_3$ und n eine Zahl von 3 bis 70 bedeuten.